# EUROPÄISCHE PATENTANMELDUNG

(11) **EP 2 087 934 A1**
(43) Veröffentlichungstag der Anmeldung: **12.08.2009**
(21) Anmeldenummer: 08151152.9
(22) Anmeldetag: 07.02.2008
(51) Int. Cl.: B01L 3/00, C12M 1/33, C12M 1/12, C12N 15/10

(54) **Verfahren und Vorrichtung zur automatisierten Prozessierung einer Probe**

(71) Anmelder: Qiagen GmbH, 40724 Hilden (DE)
(72) Erfinder: Rothmann, Thomas c/o QIAGEN GmbH, 40724 Hilden (DE); Himmelreich, Ralf c/o QUIAGEN GmbH, 40724 Hilden (DE); Laue, Thomas c/o QUIAGEN GmbH, 40724 Hilden (DE)
(74) Vertreter: Gille Hrabal Struck Neidlein Prop Roos

(57) **Zusammenfassung**

Die Erfindung betrifft ein Verfahren und eine Vorrichtung zur Prozessierung einer biologischen Probe. Prozessierung im Sinne der vorliegenden Erfindung umfasst das Binden, das Waschen sowie die Elution van Biomolekülen der biologischen Probe.
Es ist Aufgabe der Erfindung, ein Verfahren und eine Vorrichtung für die automatische Prozessierung biologischer Proben bereitzustellen, die ein relativ großes Volumen haben können und die von einem mikrofluidischen System weiter verarbeitet werden.
Zur Lösung der Aufgabe wird ein verschließbarer oder verschlossener Behälter (1) verwendet, der mit einem Filter (10) versehen ist. Eine biologische Probe wird in den Behälter gebracht. Unterhalb des Filters ist en Zu- bzw. Abfluss (4) für Flüssigkeit vorgesehen. Für de Durchführung der Prozessierung werden im Behälter befindliche Flüssigkeiten nicht nur durch den Filter hindurch abgesaugt und über den Zu- bzw. Abfluss weitergeleitet, sondern hierüber werden für die Prozessierung benötige Flüssigkeiten auch durch den Filter hindurch n den Behälter gepumpt. Da nur eine Leitung für eine automatisierte Prozessierung benötigt wird, kann der Behälter relativ einfach mit einem mikrofluidischen System verbunden werden.

## Beschreibung

Die Erfindung betrifft ein Verfahren und eine Vorrichtung zur Prozessierung einer biologischen Probe. Prozessierung im Sinne der vorliegenden Erfindung umfasst das Binden, das Waschen sowie die Elution von Biomolekülen der biologischen Probe.

Das Verarbeiten von biologischen Materialien wird z.B. auf dem Gebiet des Extrahierens und/oder des Reinigens von Biomolekülen wie Nukleinsäuren oder Proteinen angewendet. Beispielsweise basiert eine weit bekannte Methode des Reinigens der Biomoleküle auf den Schritten des Zugänglichmachens des Inhalts einer biologischen Probe ("Lyse"), des selektiven Bindens der Bestandteile des Inhalts der biologischen Probe an oder auf einem festen Stütz- oder Trägermaterial ("Binden"), des Beseitigens unerwünschter Bestandteile vom festen Stütz- oder Trägermaterial ("Waschen"), und des Lösens des gewünschten Bestandteils ("Elution").

Um ein gewünschtes Absorbieren und Desorbieren während der Aufreinigung der Biomoleküle zuzulassen, sind spezielle Filterelemente entwickelt worden, die z.B. aus Silica-Gel gebildet sind, und die einerseits porös oder Matrixartig sind, um eine Flüssigkeit durch das Filterelement hindurch gelangen zu lassen, und die andererseits eine Oberfläche haben, an die die Biomoleküle in einem spezifischen oder unspezifischen Prozess binden. In anderen Reinigungsverfahren werden Biomoleküle auf Filterelementen einfach durch den Effekt des Größenausschlusses zurückgehalten. Wenn eine Flüssigkeit, die ein Biomolekül wie z.B. eine Nukleinsäure enthält, durch das Filterelement hindurch tritt, bleiben in jedem Fall die Biomokeküle oder ein Teil davon in dem Filterelement, während der Rest durch das Filterelement hindurch gelangt. Des Weiteren wird, um das Biomolekül aus dem Filterelement zu gewinnen, eine Elutionsflüssigkeit, z.B. Nuklease-freies Wasser, zum Desorbieren des Biomoleküls auf das Filterelement geführt. Auf diese Weise wird das gewünschte Biomolekül von dem Filterelement gelöst (eluiert), und in einem Gefäß aufgefangen. Solche Filterelemente werden häufig als Membranen ausgelegt, die entweder in einzelnen Gefäßen angeordnet sind, die eine Eingangsöffnung und eine Ausgangsöffnung haben, oder die in Multiwell-Platten angeordnet sind. Die Filterelemente werden entweder mit Zentrifugen ("spin format"), oder mit auf VakuumTechnologie basierenden Apparaten prozessiert. Einzelne Gefäße mit einer Eingangsöffnung und einer Ausgangsöffnung, die eine Membran haben und die in einer Zentrifuge benutzt werden können, sind auch als Säulen, Zentifugensäul(ch)en, Filtergefäße, Chromatographiesäulen, "columns", "spin columns" oder "single spin columns" bekannt.

Im Allgemeinen sind die Vorteile des Zentrifugenverfahrens gegenüber den Vakuum basierten Methoden ein höherer Reinheitsgrad, eine höhere Konzentration und ein niedrigeres Risiko der Kreuzkontaminierung. Im Allgemeinen können die besten Resultate für die Reinigung der Biomoleküle hinsichtlich der Qualität und der Konzentration mit den Zentrifugensäulen (single spin columns) erzielt werden, die unter einem hohen Schwerefeld prozessiert werden (> 10,000 x g), da somit eine minimale Kreuzkontaminierung und eine maximale Rückgewinnung der gewünschten Substanz in Elutionsflüssikeit von der Membran möglich ist. Ein Nachteil ist jedoch die arbeitsintensive manuelle Behandlung der Zentrifugensäulen, welche das Fehlerrisiko und die Zeit für die Prozessierung erhöht, insbesondere wenn unterschiedliche Proben gleichzeitig behandelt oder verarbeitet werden sollen. Ein höherer Grad an Standardisierung und Automatisierung sowie ein höherer Durchsatz kann erzielt werden, indem man Multiwellplattenformate verwendet.

Die Firma QIAGEN GmbH aus Hilden, Deutschland, bietet ein breites Spektrum an Reinigungsprotokollen und an dafür benötigten Produkten für unterschiedliche Biomoleküle aus einer Vielzahl biologischer Proben an, die auf dem Grundprinzip des "Bind-Wash-Elute"-Protokolls basieren. Im Handel ist beispielweise das Produkt "QIAGEN QIAprep Spin Miniprep Kit" erhältlich. Zu diesem Zweck werden unterschiedliche Filtermaterialien und - vorrichtungen verwendet, wie beispielsweise beschrieben in WO 03/040364 oder US 6,277,648.

Bekannt ist also zur Prozessierung einer Probe ein Gefäß zu verwenden, welches oben offen und verschließbar ist. Am Grund des Gefäßes befindet sich eine Membran als Filterelement. Unterhalb der Membran befindet sich eine Öffnung in Form eines Stutzens, die mit beispielsweise einem Schlauch verbunden ist. Über diesen Schlauch kann Flüssigkeit aus dem Gefäß abgesaugt werden.

Zur Prozessierung wird die biologische Probe oben in das Gefäß hineingegeben. Anschließend wird die jeweils vorgesehen Lösung hineingegeben, also z. B. zunächst ein Lysepuffer, um die Probe aufzuschließen. Ist die Probe aufgeschlossen worden, so wird der Lysepuffer nach unten aus dem Gefäß herausgesaugt. Das Lysat enthält chemisch/physikalische Bedingungen, die eine Bindung der Nukleinsäure an das Trägermaterial bewirken. Die aufgeschlossene Probe (Lysat) wird anschließend gewaschen und eluiert.

Nachdem die Nukleinsäure der aufgeschlossenen Probe an die Membran gebunden oder adsorbiert wurde, werden Waschpuffer von oben in das Gefäß gegeben und nach unten aus dem Gefäß abgesaugt oder zentrifugiert. Der Waschpuffer erhält diese Bindung aufrecht, während er gleichzeitig ungewünschte Zellbestandteile aus der Membran wäscht.

Waschpuffer enthalten in der Regel Ethanol. Ethanol bewirkt eine Bindung von Nukleinsäure an eine als Filterelement wirkende Membran. Eine als Filter dienende Membran weist ein gewisses Todvolumen von beispielsweise 40µl auf. Es kann daher nicht verhindert werden, dass stets eine entsprechende Menge an Ethanol im Filter verbleibt. Ethanol trägt einerseits unerwünscht zur Bindung bei. Andererseits kann Ethanol aber auch das Ergebnis einer späteren Analyse verfälschen. Um die Nukleinsäure aus dem Gefäß nach unten entnehmen zu können und fehlerhafte Analyseergebnisse zu vermeiden, wird zunächst Ethanol entfernt, beispielsweise, indem das Gefäß zentrifugiert wird. Das Ethanol entweicht so aus der Membran und kann nach unten entnommen werden. Im Anschluss daran ist die Membran hinreichend frei von Ethanol. Mittels Vakuum und Wärmezufuhr kann Ethanol aus der Membran alternativ entfernt werden.

Um die Nukleinsäure wieder von der Membran zu lösen, wird eluiert. Dies geschieht regelmäßig mit Wasser oder einer schwach salzhaltigen wässrigen pH-stabilisierten Lösung. Im Anschluss an die Elution kann die Nukleinsäure durch die Membran hindurch aus dem Gefäß nach unten hin entnommen werden.

Problematisch bei diesem Stand der Technik ist, dass das Gefäß oder eine Vorrichtung mit diesem Gefäß immer wieder geöffnet werden muss, wie der Druckschrift "QIAamp® DNA Mini and Blood Mini Handbook, Second Edition, November 2007 (siehe http:/wwwl.qiagen.com/HB/QIAampDNAMiniAndDNABloodMiniKit_EN) zu entnehmen ist. Es kann so Probe nach außen entweichen. Es droht eine unerwünschte Kontaminierung.

Regelmäßig wird aber der Aufschluss einer biologischen Probe in einem zweiten, besser dafür geeignetem Gefäß durchgeführt, um so besonders effektiv aufschließen zu können. Ist die biologische Probe in einem zweiten Gefäß aufgeschlossen worden, so wird diese im Anschluss daran in das Gefäß mit dem Filterelement, also in eine Säule gefüllt und prozessiert. Durch das Umfüllen wird die Gefahr einer Kontamination weiter vergrößert.

Es gibt einige Publikationen zum Thema der Verarbeitung von biologischen Materialien. Beispielsweise offenbart US 6,060,022 ein automatisiertes System zur Probenverarbeitung, das einen automatisierten Zentrifugenapparat enthält. US 5,166,889 beschreibt ein Sammelsystem für Blut, in dem eine Mehrzahl von Sammelgefäßen für direkten Zugriff in einem Trägerrad positioniert sind. US 2004/0002415 beschreibt ein automatisiertes Zentrifugensystem zum automatischen Zentrifugieren von Flüssigkeiten, die biologisches Material wie z.B. Nukleinsäuren enthalten, in einer allgemeinen Zentrifuge. WO 2005/019836 beschreibt einen Apparat zum Prozessieren von Flüssigkeitsproben. WO 00138046 beschreibt ein automatisiertes Gerät zum Beladen einer Zentrifuge, bei dem Säulen der Zentrifuge überein automatisiertes Routing-System vorgestellt werden. EP 122772 beschreibt einen chemischen Manipulator zur Verwendung mit Reaktionsgefäßen. GB 2 235 639 beschreibt eine Zentrifuge mit einem den umlaufenden Kessel umgebenden Schutzmantel. Gemäß der WO 2006/042838 Al wird eine einmal verwendbare Cartridge mit einem System aus Mikrokanälen und Mikrokavitäten für einen vorgegebenen Prozessablauf nach der Probenaufnahme vorgeschlagen. Ein Apparat zum Prozessieren von biologischem Material ist aus der DE 10 2006 027 680 Al bekannt. Die DE 102005053463 Al offenbart eine Reaktionseinheit, umfassend ein Unter- und ein Oberteil zum Pipettieren von Flüssigkeiten, wobei das Unterteil aus einer Reaktionskavität mit einem durchlässigen Filtergittereinsatz besteht und das Oberteil eine Reaktionskavität darstellt, die mit dem Unterteil fixiert werden kann und eine Aussparung oder eine Hülse zur Aufnahme eines Magneten enthält. Nukleinsäure werden an magnetische Partikel gebunden. Aus dem Stand der Technik bekannte Vorrichtung zur automatisierten Durchführung einer Prozessierung weisen vielfach den Nachteil auf, dass eine technisch aufwendige Apparatur eingesetzt werden muss. Ein Beispiel für eine technisch aufwendige Apparatur stellt beispielsweise die aus der US 2006/0281094 bekannte Vorrichtung dar. Solche Vorrichtungen sind relativ teuer und erfordern relativ viel Platz. Es ist nicht möglich, eine derart aufwendige und große Apparatur in mikrofluidische, beispielsweise aus der WO 2006/042838 Al bekannte Systeme zu integrieren.

Mikrofluidische Systeme sind vorteilhaft für die Integration von komplexen Abläufen in geschlossenen Systemen (Lyse, Sample Präparation, Amplifikation, Detektion). Dieser Bedarf für geschlossene Systeme ergibt sich im diagnostischen Umfeld, bei dem Kreuzkontaminationen vermieden werden müssen. Kreuzkontaminationen können besonders schwerwiegend beim Einsatz von sehr sensitiven Detektionsmethoden (z.B. PCR - Polymerase Chain Reaktion, also ein Verfahren, mit dem in einer Kettenreaktion kleinste Mengen eines DNA-Abschnitts vervielfältigt werden können.) sein. Desweiteren werden durch die Integration der Prozeduren Bedienungsfehler vermieden.

Um alternativ oder ergänzend zur PCR die Sensitivität zu steigern, können große Probenvolumina aufgearbeitet werden. Die Aufarbeitung großer Volumen steht jedoch im Widerspruch zum Bedarf für mikrofluidische Systeme für die automatische Lyse, Prozessierung und/ oder Analyse von biologischen Proben. Es besteht daher Bedarf für Lösungen, die es erlauben, ein großes Probenvolumen über z.B. Filtration aufzuarbeiten und die isolierten Nukleinsäuren in einem kleinen Volumen über eine mikrofluidische Schnittstelle einem mikrofluidischen System zur Verfügung zu stellen. Eine derartige mikrofluidische Schnittstelle für membranbasierende Präparationsmethoden ist nicht bekannt. In der Regel wird dieses Problem in integrierten Mikrosystemen über Bead basierende Aufreinigungsverfahren gelöst.

Aus der EP 1382675 Al ist ein Extraktionsgerät mit einer Filtereinheit für die automatische Durchführung der Prozessierung (Binden-Waschen-Eluieren) von Nukleinsäuren bekannt. Dabei wird vom Anwender eine zuvor lysierte Probe auf die Filtereinheit gegeben. Der Durchlauf der Probe über die Filtermembran kann über Vakuum erfolgen. Nachfolgende Waschlösungen werden automatisiert zudispensiert und über die Membran gesaugt. Der Elutionsschritt erfolgt nach Zugabe eines Elutionspuffers in derselben Form. Diese Vorrichtung eignet sich aus nachfolgend genannten Gründen nicht für einen Anschluss an ein mikrofluidisches System, wenn das Gefäßvolumen groß sein soll.

Für die Probenaufbereitung mit filterbasierenden Spinsäulen wird stets der "Bind-Wash-Elute" Prozess gefahren. Hier werden die Flüssigkeiten ausschließlich von oben auf die Membran gegeben - so zum Beispiel auch bei der aus der EP 1382675 Al bekannten Vorrichtung - und unten am Auslass abgeführt (durch Zentrifugation oder Vakuum). Ist das Gefäß- bzw. Säulenvolumen groß, um große Flüssigkeitsmengen aufnehmen zu können, so ist diese zweckmäßig so beschaffen, dass leicht große Volumen von oben zugeführt werden können. Für die Elution in kleinen Volumen muss die Flüssigkeit jedoch exakt mittig auf die Membran pipettiert werden, um eine effiziente Elution zu erreichen. Damit ist das Zuführen von kleinen Flüssigkeitsmengen von oben für eine Anbindung an mikrofluidische Systeme nicht oder nur schwer umzusetzen und in jedem Falle nicht vorteilhaft.

Es ist Aufgabe der Erfindung, ein Verfahren und eine Vorrichtung für die automatische Prozessierung biologischer Proben bereitzustellen, die ein relativ großes Volumen haben können und die von einem mikrofluidischen System weiter verarbeitet werden.

Zur Lösung der Aufgabe wird ein verschließbarer oder verschlossener Behälter verwendet, der mit einem Filter versehen ist. Der Filter kann aus einem Silica-Gel bestehen. Eine biologische Probe wird in den Behälter gebracht und der Behälter wird vorzugsweise verschlossen. Die biologische Probe kann bereits aufgeschlossen sein. Die in den Behälter gebrachte Probe kann aber auch erst im Behälter bei Durchführung der Prozessierung aufgeschlossen werden. Unterhalb des Filters ist ein Zu- bzw. Abfluss für Flüssigkeit vorgesehen. Für die Durchführung der Prozessierung werden im Behälter befindliche Flüssigkeiten nicht nur durch den Filter hindurch abgesaugt und über den Zu- bzw. Abfluss weitergeleitet, sondern hierüber werden für die Prozessierung benötige Flüssigkeiten auch durch den Filter hindurch in den Behälter gepumpt. Da nur eine Leitung für eine automatisierte Prozessierung benötigt wird, kann der Behälter relativ einfach mit einem mikrofluidischen System verbunden werden. Es können aber auch zwei Leitungen durch den Gefäßgrund hindurchgeführt sein, wobei eine Leitung bevorzugt dem Zufluss und eine andere Leitung bevorzugt dem Abfluss dient. Allerdings hat sich eine Leitung als völlig ausreichend herausgestellt. Während der Prozessierung kann das Gefäß verschlossen bleiben. Kontaminationen, die andernfalls leicht auftreten können, werden so vermieden. Da der Behälter leicht mit einem mikrofluidischen System verbunden werden kann, kann der Behälter relativ groß sein und erst mit dem mikrofluidischen System verbunden werden, wenn dies für die Durchführung einer automatisierten Prozessierung erforderlich ist. Der Behälter kann allerdings auch fest mit einem Zu- bzw. Abfluss eines mikrofluidischen Systems verbunden sein. Ein mikrofluidisches System ist dabei ein geschlossenes System, mit dem eine biologische Probe automatisiert oder halbautomatisch präpariert und/ oder analysiert wird. Die Durchmesser der Kanäle des mikrofluidischen Systems sind relativ klein und insbesondere nicht größer als 1 mm. Ein mikrofluidisches System im Sinne der Erfindung besteht im wesentlichen aus einem flachen Substrat, welches mit Kanälen, Kammern und Ventilen versehen ist. Das Substrat ist vorzugsweise nur wenige cm breit und lang beispielsweise maximal 10 cm breit und lang und weist beispielsweise die Grundfläche einer Scheckkarte auf. Das Substrat kann bereits miniaturisierte Pumpen umfassen.

Um ein großes Volumen im Sinne der Erfindung prozessieren zu können, beträgt das Gefäßvolumen insbesondere wenigstens 150 µl, vorzugsweise wenigstens 0,5 ml, besonders bevorzugt wenigstens 1 ml. Vorzugsweise beträgt das Gefäßvolumen maximal 3 ml.

In vielen Fällen ist es möglich, eine biologische Probe im Behälter mit einem Lysepuffer, also einer Lösung aufzuschließen. Vorteilhaft wird dann der Lysepuffer ebenfalls über den Zu- bzw. Abfluss durch den Filter hindurch dem Gefäß zugeführt, um so automatisiert auch den Aufschluss der Zellen durchführen zu können. Ist der Aufschluss abgeschlossen, so wird der Lysepuffer durch den Filter hindurch über den Zu- bzw. Abfluss abgesaugt. Es verbleibt dann eine im Gefäß befindliche aufgeschlossene Probe, also Nukleinsäure, die in der Regel an dem Filter haftet.

Um eine im Gefäß befindliche biologische Probe aufschließen zu können, muss diese regelmäßig mit einem Lysepuffer hinreichend intensiv vermischt werden. Andernfalls ist ein effektives Aufschließen einer biologischen Probe nicht möglich. Versuche haben gezeigt, dass das erforderliche Mischen durch wiederholtes teilweises Absaugen von Lysepuffer und zurückpumpen in das Gefäß hinein durch den Zu- bzw. Abfluss gelingt. Als besonders wichtig hat sich dabei herausgestellt, dass der Lysepuffer dabei durch den Filter gepumpt wird. Hierdurch ergeben sich im Gefäß starke Verwirbelungen, die sich als wichtig für ein effektives Vermischen herausgestellt haben.

Wird die biologische Probe in dem Gefäß aufgeschlossen, in dem im Anschluss daran prozessiert wird, so kann ein Umfüllen der biologischen Probe von einem Gefäß in ein zweites vermieden werden. Die Gefahr einer Kontamination wird entsprechend herabgesetzt.

Im Anschluss an ein Aufschließen mit Lysepuffer wird in einer Ausführungsform der Erfindung anschließend Ethanol in das Gefäß gebracht und zwar vorzugsweise über den Zu- bzw. Abfluss. Es befindet sich zu diesem Zeitpunkt Lysepuffer und Probe im Gefäß. Ethanol trägt zwar auch ergänzend zum Aufschluss bei. Entscheidend ist allerdings, das Ethanol bewirkt, dass die Nukleinsäure verstärkt an den Filter bindet. Wird nun Lysepuffer nebst Ethanol im über den Filter sowie Zu- bzw. Abfluss vollständig abgepumpt, so bleibt die Nukleinsäure der aufgeschlossenen Probe zurück.

Sowohl das Gefäß, das Filterelement bzw. der Filter, als auch die Zuleitungen sind dann im Rahmen des Möglichen frei von Lysepuffer und Ethanol. Im Anschluss daran werden ein oder mehrere Waschpuffer in das Gefäß von unten hinein gepumpt. Der Filter nebst der aufgeschlossenen biologischen Probe wird so gewaschen und zwar wiederum vorzugsweise durch mehrfaches Zuführen und Absaugen von Waschpuffer, so dass Verwirbelungen im Gefäß erzeugt werden. Die aufgeschlossene Probe bzw. Nukleinsäure wird dadurch gereinigt und zwar besonders zuverlässig, wenn in vorgenannter Weise Verwirbelungen dabei erzeugt werden. Fette, Lipide und Proteine werden durch das Waschen entfernt.

Im Anschluss daran werden die Waschpuffer bzw. der zuletzt verwendete Waschpuffer wieder vollständig aus dem Gefäß und dem Zu- bzw. Abfluss abgesaugt.

Befindet sich im Anschluss daran Ethanol im Filter, so wird Ethanol zunächst entfernt. Vorzugsweise wird Ethanol durch Wärmzufuhr sowie durch Erzeugen eines Vakuums im Gefäß entfernt, um mechanische Bewegungen, die ein angeschlossenes mikrofluides System beschädigen könnten, zu vermeiden.

Im Anschluss daran wird Elutionsflüssigkeit, also beispielsweise Wasser oder schwach salzhaltiges Wasser von unten durch den Filter hindurch in das Gefäß gepumpt, vorzugsweise wieder zurückgesaugt und wieder hindurch gepumpt, um so nicht nur Elutionsflüssigkeit in das Gefäß zu bringen, sondern darüber hinaus Verwirbelungen zu erzeugen. Wiederum wird durch das Verwirbeln die Effizienz der Desorption der Nukleinsäuren gesteigert. Es gelingt so, die Ausbeute im Vergleich zum Stand der Technik, bei dem die diversen Flüssigkeiten bzw. Lösungen im Rahmen der Prozessierung von oben in ein Gefäß mit Filterelement eingefüllt und nach unten abgesaugt werden, zu erhöhen.

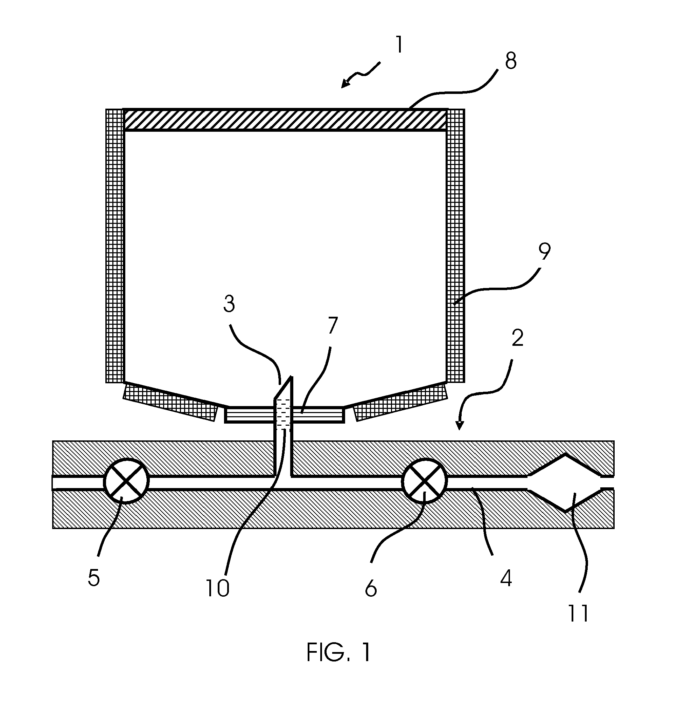

Figur 1 zeigt ein erstes Beispiel einer anspruchsgemäßen Vorrichtung und zwar einen Schnitt durch ein Gefäß 1, welches mit einem mikrofluidischen System 2 über einen Zu- bzw. Abfluss 3 verbunden ist. Der Zu- und Abfluss wird durch eine Hohlnadel 3 mit einem darin befindlichen Filter 10 gebildet. Kanäle 4 des mikrofluidischen Systems sowie die Hohlnadel 3 weisen einen Durchmesser von nicht mehr als 1 mm auf. Das mikrofluidische System umfasst darüber hinaus Ventile 5 und 6 und/ oder Pumpen, um Flüssigkeit dosiert in das Gefäß 1 pumpen oder Flüssigkeit aus dem Gefäß absaugen zu können. Der Grund des Gefäßes wird durch eine Gummimembran gebildet 7. Mit der Hohlnadel 3 wird die Membran 7 durchstochen und so das mikrofluide System mit dem Gefäß 1 verbunden. Die Hohlnadel 3 weist im Inneren eine Filtermembran 10 auf. Flüssigkeit, die aus dem Gefäß 1 in das mikrofluide System 2 gelangt oder umgekehrt, passiert zwingend den Filter 10 .

Das Gefäß 1 umfasst einen Deckel 8 mit Druckausgleich, mit dem das Gefäß oben verschlossen werden kann. Der Druckausgleich ist durch eine semipermeablen, hydrophoben Membran gewährleistet. Hierfür können PTFE Membranen (auch bekannt als GoreTex Membran) der Firma Gore benutzt werden. Durch den Druckausgleich wird ein (zu großer) Unter- oder Überdruck im Gefäß vermieden. Im unteren Bereich verjüngt sich das Gefäß trichterförmig zur Membran 7 hin, um Flüssigkeit möglichst vollständig aus dem Gefäß nach unten hin absaugen zu können. Heizelemente 9 liegen außen an dem Gefäß 1 an.

Der obere Durchmesser des Gefäßes kann einige Zentimeter, so zum Beispiel 2 cm betragen. Das Gefäß kann einige Zentimeter hoch sein, so zum Beispiel 2,5 cm. Das Gefäßvolumen kann einige ml betragen, so zum Beispiel 800 µl bis 5 ml. Als Gefäß kann eine Spin Column, so zum Beispiel der Firma Qiagen aus Hilden, Deutschland, eingesetzt werden. Als Filter kann ein Glasfasergewebe mit einer Porengröße von beispielsweise 0,7 µm bis 4 µm eingesetzt werden. Das Gefäß kann aus einem Kunststoff bestehen.

Zur Durchführung des erfindungsgemäßen Verfahrens wird zunächst eine Probe von oben in das Gefäß 1 gefüllt. Dies kann mit Hilfe einer Pipette bei geöffnetem Deckel oder über einen im Deckel vorhandenen Adapter, zum Beispiel einem Luer-Adapter geschehen. Der Deckel 8 kann aber auch mit einer Nadel durchstochen werden, um so eine biologische Probe in das Gefäß zu bringen. Zu diesem Zweck kann der Deckel ein Gummiseptum enthalten, das durchstochen wird.

Bei einem entsprechend geöffnetem ersten Ventil 5 eines angeschlossenen mikrofluidischen Systems wird bei dem in Figur 1 gezeigten Beispiel zweckmäßig über einen ersten zuführenden Kanal und über den Zu- bzw. Abfluss bzw. Hohlnadel 3 zum Beispiel der kommerziell erhältliche QIAGEN Lysepuffer AL in das Gefäß 1 gepumpt und mit der Probe intensiv vermischt, indem über Pumpen und Saugen der Flüssigkeitsstand im Gefäß 1 gehoben und abgesenkt wird. Dabei entstehen aufgrund der Membran 6 starke Turbulenzen im Gefäß. Durch das zweite, dann vorübergehend geöffnete Ventil 6 hindurch wird abgesaugt und im Wechsel damit durch das erste, zu gegebener Zeit geöffnete Ventil 5 hindurch Flüssigkeit in das Gefäß gepumpt.

Im Anschluss daran wird zum Beispiel Ethanol als Bindungmediator durch das Ventil 5 hindurch in das Gefäß 1 gepumpt. Das Lysat wird so mit dem Ethanol (EtOH) vermischt.

Nach dem Vermischen des Lysats mit dem Ethanol wird das Ventil 5 geschlossen, das Ventil 6 geöffnet und im Kanal mit dem Ventil 6 ein Unterdruck erzeugt. Dadurch wird das Lysat über den Filter 10 gezogen. Die Flüssigkeit wird so aus dem Gefäß 1 herausgesaugt. Nukleinsäuren werden dabei an den Filter 10 gebunden.

Im Anschluss daran wird zum Beispiel mit den kommerziell erhältlichen Waschpuffern AW1 und AW2 gewaschen. Und zwar wird das Ventil 6 geschlossen und das Ventil 1 geöffnet. Zuerst kann AW2 in das Gefäß gepumpt werden. Dann wird das Ventil 5 verschlossen, das Ventil 6 geöffnet und hierüber der Waschpuffer wieder aus dem Gefäß 1 abgesaugt. Im Anschluss daran wird in gleicher Weise der Waschpuffer AW1 in das Gefäß gepumpt und wieder abgesaugt. Die Reihenfolge der verwendeten Waschpuffer kann geändert werden.

Nach dem vollständigen Abpumpen der Waschpuffer durch das Ventil 6 hindurch wird das Gefäß 1 mit Hilfe der Heizelemente 9 beheizt und die erhitze Luft durch das geöffnete Ventil 6 hindurch abgepumpt. Ethanol wird so vor allem auch aus dem Filter praktisch vollständig entfernt. Ein Aufheizen auf 60°C genügt. Beträgt das Gefäßvolumen 1 ml, so genügen in der Regel jeweils 500 µl Puffer zur Durchführung des Verfahrens.

Nach Schließen des Ventils 6 und Öffnen des Ventils 5 wird zum Beispiel 50 µl des kommerziell erhältlichen Elutionspuffers AVE durch das geöffnete Ventil 5 in das Gefäß 1 hindurch gepumpt. Durch Schalten der Ventile 5 und 6 sowie durch entsprechendes Pumpen und Absaugen wird Elutionspuffer mehrfach an dem Filter 10 vorbeigeführt.

Der Elutionspuffer kann in einer Ausführungsform der Erfindung vorteilhaft in eine PCR-Kammer 11 des mikrofluidischen Systems gepumpt werden, um darin befindliche getrocknete PCR-Reagenzien zu lösen, um so schnell und einfach eine PCR durchzuführen. Das Volumen der PCR-beträgt typischerweise 50 - 100 µl und ist damit wesentlich kleiner als das Volumen des Gefäßes 1.

Bei der in Figur 1 gezeigten Ausführungsform ist es vorteilhaft möglich, das Gefäß 1 vom mikrofluiden System getrennt zu fertigen und aufzubewahren und erst zeitlich unmittelbar vor Durchführung der Präparation einer biologischen Probe miteinander zu verbinden. Bei dieser Ausführungsform muss nicht zwingend ein Deckel 8 vorgesehen sein. Statt dessen kann der Membranverschluss 7 eine Öffnung verschließen, über die das Gefäß 1 mit einer biologischen Probe gefüllt wird. Ein Mittel zur Herbeiführung eines Druckausgleichs kann statt im Bereich des Deckels 8 auch beispielsweise seitlich vorgesehen sein. Ein Mittel für die Schaffung eines Druckausgleichs im Gefäß 1 ist aber nicht zwingend erforderlich und zwar vor allem dann nicht, wenn das Gefäß stets nur teilweise mit Flüssigkeit gefüllt wird.

Es ist aber auch möglich, eine feste Verbindung, zum Beispiel eine Schraubverbindung zwischen dem Gefäß 1 und dem mikrofluidischen System 2 vorzusehen. Dann ist eine Ausgestaltung des Zu- bzw. Abflusses als vorstehende Hohlnadel überflüssig. Es genügt ein normales Röhrchen bzw. Kanal.

Zum Nachweis der Machbarkeit wurde genomische DNA aus 200 µl CPDA-Blut (Blutbeutel) mit dem kommerziell erhältlichen QlAamp Mirco Kit der Firma QIAGEN aus Hilden, Deutschland, präpariert. Das erfindungsgemäße Verfahren wurde folgendermaßen durchgeführt.
- Die QlAamp MinElute Spin Column wurde auf einen Luer Drei-Wege Hahn gesteckt und diente als Gefäß im Sinne der vorliegenden Erfindung.
- 20 µl Proteinase K und 200 µl Blut wurden von "oben" in die Säule gefüllt.
- Durch einen Kanal des Luer Hahns wurde von "unten" der Säule 200µl Puffer AL zugeführt.
- Durch Vor- und Zurückbewegen des Spritzenkolbens einer angeschlossenen 1 ml Spritze wurden die Flüssigkeiten innerhalb der Säule und in der Spritze vermischt.
- Der Säuleninhalt wurde nun in ein Eppendorf-Tube überführt und 15 min bei 56°C inkubiert. Dieser Inkubationsschritt kann auch alternativ in der Säule durchgeführt werden, in dem die Säule mit einem weiblichen Luer-Stopfen verschlossen wird und in einem Eppendorf Thermomixer (gehalten von dem "Collection Micro-Tube; im Kit enthalten) inkubiert wird. Hierbei muss der Wärmeeintrag wegen der doppelten Wandung optimiert werden. Prinzipiell wurde gezeigt, dass auch diese Variante des Protokolls funktioniert.
- Nach Inkubation und Rückführung des Lysats in die Spin-Column wurde über den Luer Hahn 200 µl Ethanol von unten der Säule zugeführt.
- Durch Vor- und Zurückbewegen des Spritzenkolbens wurden die Flüssigkeiten innerhalb der Säule vermischt.
- Der Säuleninhalt wurde nun durch eine großvolumige Spritze abgesaugt. Hierbei wird die DNA an die Silikamembran gebunden
- Nun werden 500 µl Waschpuffer AW1 mit einer Spritze von unten in die Säule hinein zugeführt, und durch Vor- und Rückbewegen des Spritzenkolbens wird die Membran gewaschen. Der Säuleninhalt wurde nun durch eine großvolumige Spritze abgesaugt.
- Der vorangegangene Schritt wird mit dem Waschpuffer AW2 wiederholt.
- Nach Entfernung des Waschpuffers wird die Membran unter Vakuum von unten getrocknet (4 min bei -800 mbar).
- Die DNA wird nun mit 50 µl Puffer AE eluiert. Hierzu wird das Volumen mit einer 1 ml Spritze zugeführt, und durch Vor- und Zurückbewegen des Spritzenkolbens für 30 sec wird die DNA aus der Membran eluiert. Das gesamte Eluat wird wieder in die Spritze zurückgesaugt und in ein 1,5 ml Reaktionsgefäß Microtube überführt, um manuell quantitative und qualitative Analysen durchführen zu können.

Bei Referenzproben wurde das Protokoll gemäß den im QlAamp Blood Mini Kit Handbuch der Firma Qiagen aus Hilden, Deutschland, beschriebenen Vorgaben durchgeführt.

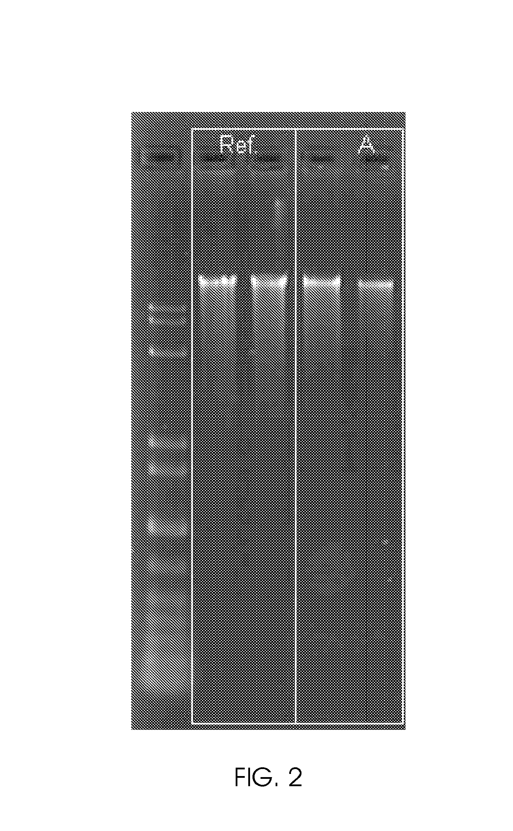

Nach der Präparation wurden die Nukleinsäuren auf einem Agarosegel elektrophoretisch aufgetrennt. Nach der Auftrennung wurden die Nukleinsäurefragmente in einem Ethidiumbromid-haltiges Wasserbad eingefärbt, auf einer UV-Leuchtplatte zum Leuchten gebracht und durch ein Fotodokumentationssystem abgelichtet. Die genomische DNA ist als helle Bande im oberen Drittel der Figur 2a zu sehen.

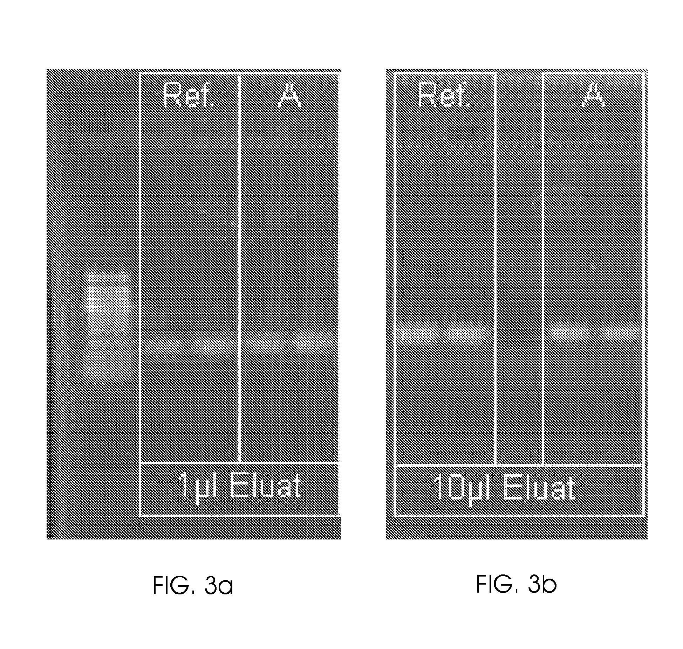

Die Figuren 2 und 3a und 3b zeigen die Analysedaten der beschriebenen Versuche. Figur 2 bezieht sich auf Agarose Gel. Die Stärke, mit anderen Worten die Leuchtintensität (Anzahl und Fläche weisser Pixel) einer Bande in der Figur 2 ist ein Maß für die Quantität. Die Lage und die Ausprägung der Banden ist ein Maß für die Qualität. Das Gel bestätigt, dass vergleichbare Konzentrationen unabhängig davon erhalten werden, ob gemäß dem aus dem Stand der Technik bekannten Handbuch oder gemäß dem anspruchsgemäßen Verfahren vorgegangen wird.

Die Figuren 3a und 3b beziehen sich auf Agarose Gel nach PCR. Die Banden in der Spalte "Ref." beziehen sich auf die nach dem Stand der Technik durchgeführten Vergleichsversuche. Die Banden in der Spalte "A" beziehen sich auf die erfindungsgemäß durchgeführten Versuche. Es wurden unterschiedliche Volumina an Eluat der Proben in eine PCR eingesetzt, nämlich 1µl Eluat (Figur 3a) und 10µl Eluat (Figur 3b) und danach auf einem Agarosegel aufgetrennt. Die Banden in der Bildmitte (vertikal gesehen) zeigen die amplifizierte Nukleinsäure. Es wurden praktisch keine Unterschiede zwischen einer Bande, die aus einem Vergleichsversuch erhalten wurde, und einer Bande, die erfindungsgemäß erhalten wurde, festgestellt. Hieraus folgt erstens, dass die gewonnene Nukleinsäure erfolgreich für PCR Reaktion benutzt werden können. Zweitens zeigt der Erfolg bei 10 µl Eluatinputvolumen, dass die Qualität der Nukleinsäure nicht inhibierend auf die PCR wirkt. Je nach Präparationsmethode kann es vorkommen, das bei 10 µl Eluatinputvolumen die PCR nicht funktioniert, weil inhibitorische Moleküle als Verunreinigung im Eluat vorliegen und bei erhöhtem Eluatinputvolumen in die PCR drastische Auswirkungen hervorrufen.

Die durchgeführten Versuche zeigen also, dass eine erfindungsgemäß im Vergleich zum Stand der Technik veränderte Führung der Flüssigkeiten bei Spin-Columns möglich ist, ohne Nachteile in Bezug auf die erhaltenen Ergebnisse in Kauf nehmen zu müssen. Die veränderte Führung der Flüssigkeiten bei Spin-Columns eröffnet neue Möglichkeiten in der Umsetzung von Membrantechnologie zur Nukleinsäurereinigung im mikrofluidischen Umfeld.

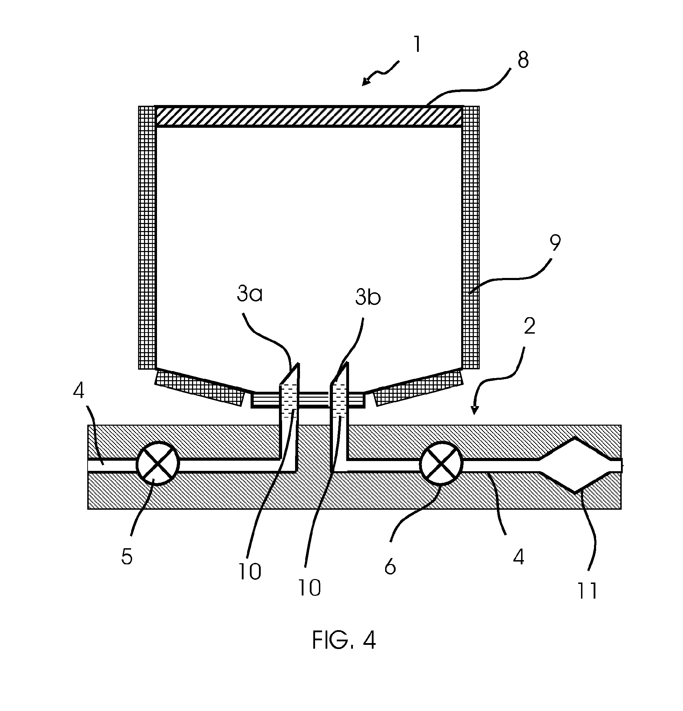

Figur 4 zeigt eine alternative Ausführungsform mit einem Zufluss 3a und einem Abfluss 3b, die jeweils als Hohlnadel ausgestaltet sind. Zufluss und Abfluss liegen nahe beieinander. Daher ist es bei dieser Ausgestaltung ebenfalls sehr leicht möglich, das relativ große Gefäß (1) mit einem mikrofluidischen System zu verbinden, um so ein großes Gefäßvolumen bereitzustellen. Das Gefäßvolumen ist insbesondere dann relativ groß, wenn es wenigstens doppelt so groß, vorzugsweise wenigstens dreimal so groß wie das Volumen der größten Kammer ist, die in das mikrofluidische System integriert ist. Also ist dann beispielsweise das Volumen des Gefäßes 1 wenigstens doppelt, vorzugsweise wenigstens dreimal so groß wie das Volumen der gezeigten PCR-Kammer 11.

Aus Figur 4 wird deutlich, dass nicht zwingend derselbe Filter für den Zu- und Abfluss eingesetzt werden muss. Der Zufluss muss noch nicht einmal zwingend einen Filter umfassen, um das relativ große Gefäß (1) geeignet mit einem mikrofluidischen System verbinden zu können. Allerdings handelt es sich dann um eine verschlechterte Ausführungsform der Erfindung, da die Erzeugung von erwünschten Turbulenzen schwieriger ist.

Anstelle von Gummimembranen können auch andere aus elastischem Material bestehende Membranen eingesetzt sein. Von besonderer Bedeutung ist dabei lediglich, dass eine solche Membran von einer Nadel durchstoßen werden kann und im Anschluss daran die Nadel außen flüssigkeitsdicht mit der Membran verbunden ist. Kann eine Membran elastisch ausgedehnt werden, so kann diese für einen Druckausgleich sorgen. Insbesondere ist diese für eine relativ großflächige Membran von Bedeutung, die das Gefäß nach oben hin begrenzt.

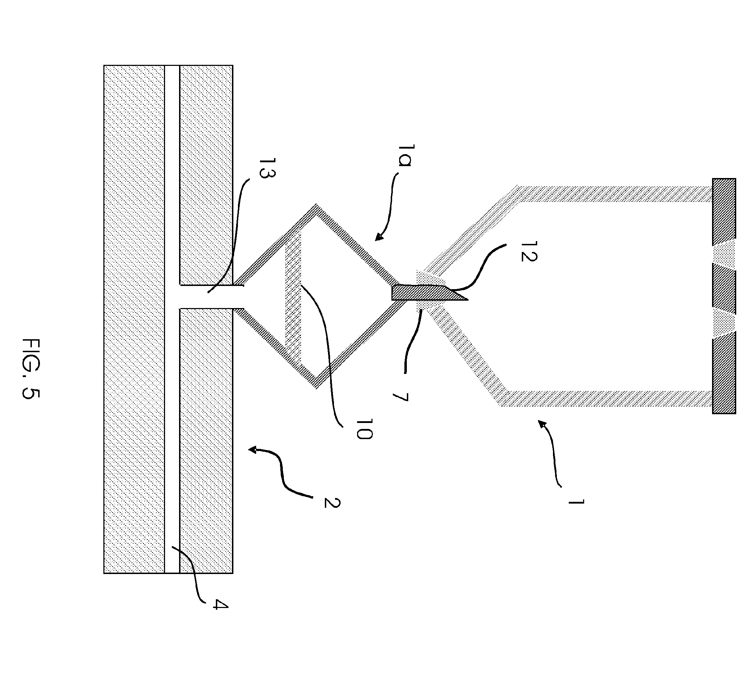

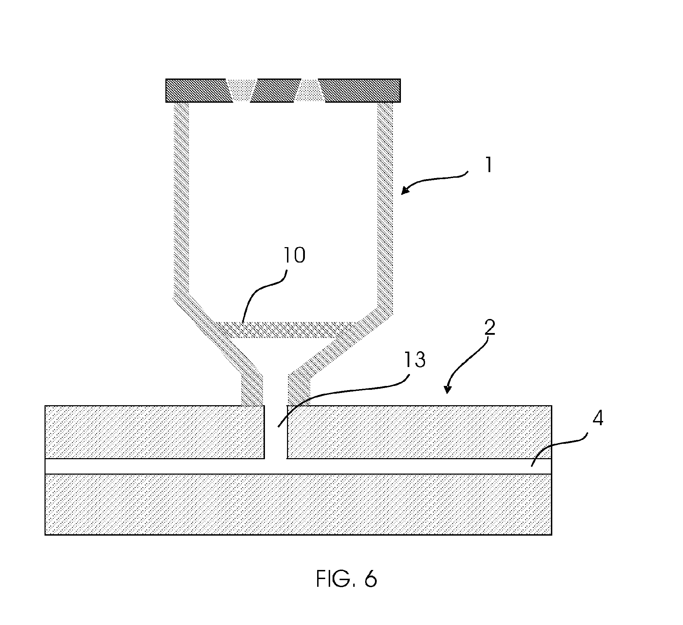

Figur 5 zeigt ein weiteres Beispiel einer Vorrichtung zur Durchführung des erfindungsgemäßen Verfahrens. Mit einem mikrofluidischen System 2 ist eine Kammer 1a fest verbunden. Die Kammer 1a ist vorzugsweise relativ klein zu einem zugehörigen Gefäß 1. Insbesondere ist das Volumen des Gefäßes 1 wenigstens doppelt so groß wie das Volumen der Kammer 1a. In der Kammer 1a befindet sich ein Filter 10. Unterhalb des Filters 10 gibt es einen Zufluss bzw. Abfluss 13, über die Flüssigkeit aus dem mikrofluidischen System 2 in die Kammer 1a geleitet werden kann und umgekehrt. Oberhalb des Filters 1 0 weist die Kammer 1a eine Hohlnadel 1 2 auf, mit der ein Gummiseptum oder eine Membran 7 des Gefäßes 1 durchstochen werden kann, um so eine Verbindung zwischen der Kammer 1a und dem Gefäß 1 zu schaffen. Diese Ausführungsform weist gegenüber der in Figur 1 gezeigten Ausführungsform den Vorteil auf, dass das Filterelement 10 leicht eine relativ große Oberfläche aufweisen kann, da sich das Filterelement nicht in einer dünnen Hohlnadel befindet, sondern in einem sehr viel breiteren Gefäß. Die größere Filteroberfläche wirkt sich günstig auf die Durchführung des Verfahrens aus. So steht mehr Fläche zur Verfügung, um Turbulenzen und damit eine gute Durchmischung zu erzeugen. Die in Figur 5 gezeigte Ausführungsform weist gegenüber einer in der nachfolgenden Figur 6 gezeigten Ausführungsform den Vorteil auf, dass ein auf dem mikrofluidischen System fest aufgebrachtes Gefäß bzw. Kammer relativ klein sein kann. Die gute Handhabbarkeit des mikrofluidischen System wird so durch das aufgebrachte Gefäß bzw. die aufgebrachte Kammer weniger beeinträchtigt. Im übrigen funktioniert die in Figur 5 gezeigte Ausführungsform in gleicher Weise wie die in Figur 1 gezeigte Ausführungsform.

Figur 6 zeigt den Fall, dass das Gefäß 1 fest mit dem mikrofluidischen System 2 über einen Zufluss bzw. Abfluss 13 verbunden ist. Im Gefäß 1 befindet sich nahe bei dem Zu- bzw. Abfluss 1 3 ein relativ großflächiges Filterelement 10.

Das anspruchsgemäße Verfahren kann auch auf Proteine übertragen werden.

## Patentansprüche

1. Verfahren für die Prozessierung einer biologischen Probe in einem Gefäß (1) mit einem Filterelement (1 0) und einer im Gefäß oder Kammer (1, 1a) befindlichen biologischen Probe, **dadurch gekennzeichnet, dass** für die Prozessierung verwendete Puffer durch einen Zu- bzw. Abfluss (3, 13) sowie durch den Filter (10) hindurch in das Gefäß und/ oder Kammer (1, 1a) geleitet werden.

2. Verfahren nach Anspruch 1 , bei dem die Puffer über ein mikrofluidisches System (2) in das Gefäß (1) gepumpt werden sowie über das mikrofluidisches System (2) aus dem Gefäß und/ oder Kammer (1, 1a) abgesaugt werden.

3. Verfahren nach dem vorhergehenden Anspruch, bei dem die Durchmesser der Kanäle des mikrofluidischen Systems nicht größer als 1 mm sind.

4. Verfahren nach einem der vorhergehenden Ansprüche, bei dem das Füllvolumen des Gefäßes (1) wenigstens 150 µl, vorzugsweise 1 ml beträgt.

5. Verfahren nach einem der vorhergehenden Ansprüche, bei dem ein Lysepuffer für das Aufschließen der im Gefäß und/ oder Kammer (1, 1a) befindlichen biologischen Probe durch den Filter (1 0) hindurch in das Gefäß und/ oder die Kammer (1, 1a) gepumpt wird.

6. Verfahren nach einem der vorhergehenden Ansprüche, bei dem der Flüssigkeitspegel eines im Gefäß und/ oder in der Kammer (1, 1a) befindlichen Puffers mehrmals gehoben und gesenkt wird, indem der Puffer teilweise durch den Filter (10) hindurch in das Gefäß und/ oder in die Kammer (1, 1a) gepumpt und teilweise hierüber herausgesaugt wird.

7. Verfahren nach einem der vorhergehenden Ansprüche, bei dem Ethanol durch den Filter (10) hindurch in das Gefäß und/ oder Kammer (1, 1a) mit einer darin befindlichen, aufgeschlossenen Probe gepumpt und anschließend über den Filter aus dem Gefäß (1) und/ oder aus der Kammer (1, 1a) herausgesaugt wird.

8. Verfahren nach einem der vorhergehenden Ansprüche, bei dem ein Waschpuffer durch den Filter (10) hindurch in das Gefäß und/ oder Kammer (1, 1a) mit einer darin befindlichen, aufgeschlossenen Probe gepumpt und anschließend über den Filter aus dem Gefäß und/ oder Kammer (1, 1a) herausgesaugt wird.

9. Verfahren nach einem der vorhergehenden Ansprüche, bei dem Ethanol durch Erhitzen des Gefäßes und/ oder Kammer (1, 1a) und/oder Absaugen aus dem Gefäß entfernt wird.

10. Verfahren nach einem der vorhergehenden Ansprüche, bei dem eine Elutionsflüssigkeit durch den Filter (1 0) hindurch in das Gefäß (1) mit einer darin befindlichen, aufgeschlossenen Probe gepumpt und/ oder Kammer (1a) und anschließend über den Filter aus dem Gefäß und/oder Kammer (1, 1a) herausgesaugt wird.

11. Verfahren nach einem der vorhergehenden Ansprüche, bei dem das Gefäß (1) über eine Hohlnadel (3, 3a, 3b) mit einem mikrofluidischen System (2) verbunden wird.

12. Vorrichtung zur Durchführung eines Verfahrens nach einem der vorhergehenden Ansprüche mit einem mikrofluidischem System (2) und einem damit über einen Filter (10) flüssigkeitsleitend verbundenem Gefäß (1), wobei das Volumen des Gefäßes größer, vorzugsweise sehr viel größer im Vergleich zu Volumen von Kammern (1a, 11) ist, die Teil des mikrofluidischen Systems sind.

13. Vorrichtung nach dem vorhergehenden Anspruch, bei der die Durchmesser der Kanäle des mikrofluidischem System (2) nicht größer als 1 mm sind.

14. Vorrichtung nach einem der beiden vorhergehenden Ansprüche, bei der das Gefäßvolumen des Gefäßes (1) wenigstens 150 µl, vorzugsweise wenigstens 1 ml beträgt.

15. Vorrichtung nach einem der drei vorhergehenden Ansprüche, bei der das Gefäß (1) auf das Substrat eines mikrofluidischen Systems (2) aufgesetzt ist.

16. Mikrofluidisches System für eine Vorrichtung nach einem der vorhergehenden Ansprüche mit einem Substrat und darin befindlichen und/ oder darauf aufgebrachten Kanälen (4), Ventilen (5, 6), Kammern (1a, 11) und/ oder Pumpen, **gekennzeichnet durch** eine mit wenigstens einem Kanal (4) verbundene Hohlnadel (3, 3a, 3b) für das Durchstoßen des Grundes (7) eines Gefäßes (1).

17. Mikrofluidisches System nach dem vorhergehenden Anspruch mit einem Filter (10) in einer Hohlnadel (3, 3a, 3b).

18. Mikrofluidisches System nach einem der beiden vorhergehenden Ansprüche, wobei die Durchmesser der Kanäle des Systems nicht größer als 1 mm sind.
